# EUROPEAN PATENT SPECIFICATION

(11) **EP 0 515 145 B1**
(45) Date of publication and mention of the grant of the patent: **10.07.1996**
(21) Application number: 92304531.4
(22) Date of filing: 19.05.1992
(51) Int. Cl.: C07H 13/04, C07H 9/04, C07H 5/02

(54) **Continuous process for the preparation of sucrose 6-esters**
Kontinuierliches Verfahren zur Herstellung von Sucrose-6-Estern
Procédé continu de préparation de sucrose-6-esters

(30) Priority: 21.05.1991 GB 9110821
(43) Date of publication of application: 25.11.1992
(73) Proprietor: TATE & LYLE PUBLIC LIMITED COMPANY, London, EC3R 6DQ (GB)
(72) Inventor: Sankey, George Henry, Earley, Reading, Berkshire RG6 2TQ (GB)
(74) Representative: Ablewhite, Alan James

(56) References cited:
- EP-A- 0 260 979
- EP-A- 0 423 745
- LASZLO P.: 'PREPARATIVE CHEMISTRY USING SUPPORTED REAGENTS.', 1987, ACADEMIC PRESS, INC., SAN DIEGO
- DORFNER K.: 'ION EXCHANGERS.', 1991, WALTER DE GRUYTER, BERLIN

## Description

This invention relates to an improved process for the preparation of sucrose 6-esters, which are key intermediates in one route to the production of sucralose (1,6-dichloro-1,6-dideoxy-β-D-fructofuranosyl 4-chloro-4-deoxy-α-D-galactopyranoside). Sucralose is a high intensity sweetener having a sweetness of several hundred times that of sucrose which is disclosed in our British Patent No. 1,543,167.

The preparation of sucralose involves the introduction of chlorine atoms into the 1′-and 6′- positions (i.e. displacement of two of the three primary hydroxyl groups) and at the 4-position (i.e. displacement of a secondary hydroxyl group). The third primary hydroxyl group, at the 6-position, must remain unaffected.

One important route to sucralose involves the preparation and subsequent chlorination of 2,3,6,3′ ,4′-penta-O-acetyl sucrose in which the three hydroxyl groups to be displaced by chlorine atoms are unprotected while all the other hydroxyl groups are protected (see, for example, US Patent No. 4,362,869 and UK Patent GB 2 065 648 B).

An alternative, simpler route involves the preparation of a sucrose 6-ester which can be selectively chlorinated at the 4-, 1′- and 6′- positions and then de-esterified to provide sucralose. One such method of preparing sucrose 6-esters and converting them to sucralose is disclosed in our UK Patent GB 2 079 749 B, but this method produces a mixture of acylated sucrose derivatives with substituents at one or more of the primary positions, with a major proportion of the 6-ester.

A more selective method of preparing sucrose 6-esters, disclosed in our UK Patent GB 2 195 632 B, is based upon the preparation of a sucrose alkyl 4,6-orthoester by reacting sucrose with a trialkyl orthoacylate in the presence of an acid catalyst in an inert organic solvent, followed by hydrolysis of the orthoester to provide a mixture of sucrose 4- and 6-esters, which are then isomerised to provide a high yield of the required sucrose 6-ester. Ketene acetals, such as 1,1-dimethoxyethene, are useful alternatives to trialkyl orthoacylates in the preparation of sucrose alkyl 4,6-orthoesters, as disclosed in our British Patent Application (No. 9110931.4) entitled Process for the preparation of Sucrose 6-esters, filed on 21st May 1991.

The method of GB 2 195 632 B is more efficient than that of GB 2 079 749 B, but involves several stages, each utilising a different acid or base which must be removed.

We have now found that the method of GB 2 195 632 B can be operated in a semi-continuous or continuous mode, using an acidic ion exchange resin to catalyse the preparation of the sucrose alkyl 4,6-orthoester and to hydrolyse it to a mixture of sucrose 4- and 6-esters, and then treating the hydrolysate with a base to convert the sucrose 4-ester to sucrose 6-ester. The process can be run continuously to produce a mixture of sucrose 4- and 6-esters, without the need to add or remove acid.

Thus, according to the present invention, there is provided a continuous process for the preparation of a sucrose 6-ester by:
(i) reacting sucrose with a trialkyl orthoester or with a ketene acetal in an inert organic solvent in the presence of an acid catalyst to provide a sucrose alkyl 4,6-orthoester,
(ii) treating the sucrose alkyl 4,6-orthoester under mild aqueous acidic conditions to provide a mixture of sucrose 4- and 6-monoesters,
(iii) treating the mixture of esters with a base to convert the sucrose 4-ester into sucrose 6-ester, and
(iv) neutralising the solution and isolating the sucrose 6-ester,
characterised in that a strongly acidic macroreticular ion exchange resin is used at ambient temperature to catalyse preparation of the sucrose alkyl 4,6-orthoester and to effect its hydrolysis in step (ii), and in that a solution of sucrose and a trialkyl orthoester or a ketene acetal in an inert organic solvent is passed through a column of macroreticular acid resin to provide an eluate containing a sucrose alkyl 4,6-orthoester, water being continuously added to the eluate before passing the aqueous solution through a second column of acidic ion exchange resin to provide a mixture of sucrose 4- and 6- monoesters.

In general, the amount of trialkyl orthoester or ketene acetal used in step (i) can vary from about 1 to 2 ME per ME of sucrose, preferably from 1.1 to 1.5 ME.

Amongst the wide range of acidic ion exchange resins available, we have found that the most useful type to act as catalyst for preparation of the sucrose alkyl 4,6-orthoester is a macroreticular acid resin.

Choice of an acidic ion exchange resin for hydrolysis of the mixture of sucrose 4- and 6-esters is much less critical, and selection of the resin for this step is mainly a matter of cost and convenience.

Stages (i) and (ii) of the process are run continuously by passing a solution of sucrose and the reagent in an inert organic solvent such as dimethylformamide through a column of macroreticular acid resin, continuously adding water to the eluate containing the sucrose alkyl 4,6-orthoester, and passing the aqueous solution through a second column of acidic ion exchange resin to effect the hydrolysis.

We have found that Amberlyst 15(H⁺) macroreticular resin is suitable for the first stage and that it is convenient to use the same resin for stage (ii).

Conversion of the sucrose 4-ester to sucrose 6-ester can be carried out by adding a water soluble base such as potassium carbonate or sodium carbonate. Alternatively, an organic base such as tertiary butylamine can be used.

Use of a readily water soluble base such as potassium carbonate lends itself to continuous operation more easily than a less soluble base such as calcium hydroxide. Thus, the eluate from stage (ii) can be collected in a holding tank, into which the base is metered to provide the appropriate concentration for migration of the ester group from the 4- to the 6- position.

The base must be neutralised before concentration and isolation of the sucrose 6-ester, in order to prevent degradation of the product. Neutralisation can be effected by addition of a suitable acid or, preferably, by passage through an acidic ion exchange resin to give a stream free from inorganic salts.

The sucrose 6-ester can be isolated by concentrating the neutralised solution to a syrup, dissolving the syrup in methanol, seeding, and collecting the crystalline product. After washing, e.g. with methanol, and drying, the sucrose 6-ester can be stored until required.

Sucrose 6-esters prepared according to the present invention can be used as intermediates for the production of sucralose. Thus, in a further embodiment of the present invention there is provided a process for the production of sucralose, by preparing a sucrose 6-ester, reacting the sucrose 6-ester with a chlorinating agent capable of selectively chlorinating the 4-, 1′- and 6′ positions, optionally peresterifying the sucralose 6-ester so formed, de-esterifying the sucralose ester, and recovering sucralose, in which the sucrose 6-ester is prepared by:
(i) passing a solution of sucrose and a trialkyl orthoester or a ketene acetal in an inert organic solvent through a macroreticular acid resin to provide an eluate containing a sucrose alkyl 4,6-orthoester,
(ii) continuously adding water to the eluate and passing the mixture through an acidic ion exchange resin to hydrolyse the sucrose alkyl 4,6-orthoester and to provide an eluate containing a mixture of sucrose 4- and 6- monoesters,
(iii) treating the mixture of esters with a base to convert the sucrose 4-ester to sucrose 6-ester and (iv) neutralising the solution and isolating the sucrose 6-ester.

The invention is further illustrated by the following Example.

### Example 1 Preparation of sucrose 6-acetate

A solution of sucrose (500 g) and trimethyl orthoacetate (225 ml; 1.2 ME) in dimethylformamide (2500 ml) was passed down a column of Amberlyst 15(H⁺) resin (25 g) at ambient temperature at a rate of 10 g/minute. Water was added continuously to the eluate issuing from the base of the column at a rate of 0.065 g water/g eluate and the diluted eluate was passed through a heat exchanger to cool the temperature from about 40° (heat of dilution) to about 25°.

The cooled solution from the heat exchanger was then passed down a second column of Amberlyst 15(H⁺) resin (25 g) at a rate of 10.65g/minute (i.e. at a rate which allowed the two columns to be run continuously in sequence).

A sample of the eluate from the second column (1851 g; equivalent to an input of 300 g of sucrose) was removed and treated with an aqueous 2% w/v solution of potassium carbonate (K₂CO₃. 1.5H₂O; 37 ml). After one hour the solution was neutralised with dilute hydrochloric acid (0.5 molar; 16.2 ml) and then concentrated to a syrup (451.2 g) under reduced pressure at 50°. Analysis of the syrup showed a yield of crude sucrose 6-acetate of about 75% with sucrose (about 15%) as the main impurity.

The syrup was dissolved in hot methanol (600 ml), allowed to cool to 40° and then seeded with crystalline sucrose 6-acetate (1.5 g). Crystallisation was allowed to proceed overnight at ambient temperature and then the product was collected, washed with methanol (300 ml) and dried in vacuo at 40°. Yield 219.4 g; 50% molar; analysis: sucrose 6-acetate 82.8%, sucrose 4-acetate 0.5%, sucrose 4.7%, sucrose di-acetate 2.4%, methanol of crystallisation 7%.

Similar results can be obtained by using 1,1-dimethoxyethene (150 ml) in place of the trimethyl orthoacetate.

## Claims

1. A continuous process for the preparation of a sucrose 6-ester by:
(i) reacting sucrose with a trialkyl orthoester or with a ketene acetal in an inert organic solvent in the presence of an acid catalyst to provide a sucrose alkyl 4,6-orthoester,
(ii) treating the sucrose alkyl 4,6-orthoester under mild aqueous acidic conditions to provide a mixture of sucrose 4- and 6-monoesters,
(iii) treating the mixture of esters with a base to convert the sucrose 4-ester into sucrose 6-ester, and
(iv) neutralising the solution and isolating the sucrose 6-ester,
characterised in that a strongly acidic macroreticular ion exchange resin is used at ambient temperature to catalyse preparation of the sucrose alkyl 4,6-orthoester and to effect its hydrolysis in step (ii), and in that a solution of sucrose and a trialkyl orthoester or a ketene acetal in an inert organic solvent is passed through a column of macroreticular acid resin to provide an eluate containing a sucrose alkyl 4,6-orthoester, water being continuously added to the eluate before passing the aqueous solution through a second column of acidic ion exchange resin to provide a mixture of sucrose 4- and 6- monoesters.

2. A process according to Claim 1, in which the amount of trialkyl orthoester or ketene acetal used in step (i) is from about 1 to 2 mole equivalents (ME) per ME of sucrose.

3. A process according to Claim 2, in which the amount of trialkyl orthoester or ketene acetal used in step (i) is from about 1.1 to 1.5 ME per ME of sucrose.

4. A process according to any of Claims 1 to 3, in which the base used to convert the sucrose 4-ester into the sucrose 6-ester is a water soluble base or an organic base.

5. A process according to any of Claims 1 to 4, in which the mixture of sucrose 4- and 6- monoesters thus produced is collected in a holding tank into which is metered a water soluble base to effect conversion of the sucrose 4-ester into the sucrose 6-ester.

6. A process according to any of Claims 1 to 5, in which the base is neutralised after conversion of the sucrose 4-ester into the sucrose 6-ester by passing the basic solution through an acidic ion exchange resin.

7. A method for the production of sucralose, comprising preparing a sucrose 6-ester, reacting the sucrose 6-ester with a chlorinating agent capable of selectively chlorinating the 4-, 1'- and 6' positions, optionally peresterifying the sucralose 6-ester so formed, de-esterifying the sucralose ester,s and recovering sucralose, in which the sucrose 6-ester is prepared by:
(i) passing a solution of sucrose and a trialkyl orthoester or a ketene acetal in an inert organic solvent through a macroreticular strong acid resin to provide an eluate containing a sucrose alkyl 4,6-orthoester,
(ii) continuously adding water to the eluate and passing the mixture through an acidic ion exchange resin to hydrolyse the sucrose alkyl 4,6-orthoester and to provide an eluate containing a mixture of sucrose 4- and 6- monoesters,
(iii) treating the mixture of esters with a base to convert the sucrose 4-ester to sucrose 6-ester, and (iv) neutralising the solution and isolating the sucrose 6-ester.

## Patentansprüche

1. Kontinuierliches Verfahren zur Herstellung eines Sucrose-6-Esters durch:
(i) Umsetzung von Sucrose mit einem Trialkylorthoester oder mit einem Ketenacetal in einem inerten organischen Lösungsmittel in Anwesenheit eines sauren Katalysators, um einen Sucrosealkyl-4.6-Orthoester zu liefern,
(ii) Behandlung des Sucrosealkyl-4,6-Orthoesters unter milden wäßrigsauren Bedingungen, um ein Gemisch von Sucrose-4- und -6-Monoester zu liefern,
(iii) Behandlung des Gemisches von Estern mit einer Base, um den Sucrose-4-Ester in Sucrose-6-Ester umzuwandeln, und
(iv) Neutralisierung der Lösung und Isolierung des Sucrose-6-Esters,
dadurch gekennzeichnet, daß ein stark saures makroporöses Ionenaustauscherharz bei Umgebungstemperatur verwendet wird, um die Herstellung des Sucrosealkyl-4.6-Orthoesters zu katalysieren und dessen Hydrolyse im Schritt (ii) zu bewirken, und dadurch, daß man eine Lösung von Sucrose und einem Trialkylorthoester oder einem Ketenacetal in einem inerten organischen Lösungsmittel durch eine Säule mit einem makroporösen sauren Harz laufen läßt, um ein Eluat zu liefern, das einen Sucrosealkyl-4,6-Orthoester enthält, wobei dem Eluat kontinuierlich Wasser zugesetzt wird, bevor man die wäßrige Lösung durch eine zweite Säule mit einem sauren Ionenaustauscherharz laufen läßt, um ein Gemisch von Sucrose-4- und -6-Monoester zu liefern.

2. Verfahren nach Anspruch 1, bei dem die Menge des im Schritt (i) verwendeten Trialkylorthoesters oder Ketenacetals von etwa 1 bis 2 Moläquivalente (MÄ) pro MÄ Sucrose beträgt.

3. Verfahren nach Anspruch 2, bei dem die Menge des im Schritt (i) verwendeten Trialkylorthoesters oder Ketenacetals von etwa 1,1 bis 1,5 MA pro MÄ Sucrose beträgt.

4. Verfahren nach einem der Ansprüche 1 bis 3, bei dem die zur Umwandlung des Sucrose-4-Esters in den Sucrose-6-Ester verwendete Base eine wasserlösliche Base oder eine organische Base ist.

5. Verfahren nach einem der Ansprüche 1 bis 4, bei dem das so hergestellte Gemisch von Sucrose-4- und -6-Monoester in einem Vorratsbehälter gesammelt wird, in den eine wasserlösliche Base zudosiert wird, um die Umwandlung des Sucrose-4-Esters in den Sucrose-6-Ester zu bewirken.

6. Verfahren nach einem der Ansprüche 1 bis 5, bei dem die Base nach der Umwandlung des Sucrose-4-Esters in den Sucrose-6-Ester neutralisiert wird, indem man die basische Lösung durch ein saures Ionenaustauscherharz laufen läßt.

7. Verfahren zur Herstellung von Sucralose, umfassend die Herstellung eines Sucrose-6-Esters, Umsetzung des Sucrose-6-Esters mit einem Chlorierungsmittel, das fähig ist, selektiv die 4-, 1'- und 6'-Position zu chlorieren, gegebenenfalls Perveresterung des so gebildeten Sucralose-6-Esters, Entesterung des Sucraloseesters und Rückgewinnung der Sucralose, wobei der Sucrose-6-Ester hergestellt wird durch:
(i) Durchlaufenlassen einer Lösung von Sucrose und einem Trialkylorthoesters oder einem Ketenacetal in einem inerten organischen Lösungsmittel durch ein makroporöses stark saures Harz, um ein Eluat zu liefern, das einen Sucrosealkyl-4,6-Orthoester enthält,
(ii) kontinuierliche Zugabe von Wasser zu dem Eluat und Durchlaufenlassen des Gemisches durch ein saures Ionenaustauscherharz, um den Sucrosealkyl-4,6-Orthoester zu hydrolysieren und ein Eluat zu liefern, das ein Gemisch von Sucrose-4- und -6-Monoester enthält,
(iii) Behandlung des Gemisches von Estern mit einer Base, um den Sucrose-4-Ester in den Sucrose-6-Ester umzuwandeln und
(iv) Neutralisierung der Lösung und Isolierung des Sucrose-6-Esters.

## Revendications

1. Procédé continu pour la préparation de 6-ester de saccharose, comprenant les étapes de:
(i) réaction du saccharose avec un trialkyle orthoester ou avec un cétène acétal dans un solvant organique inerte en présence d'un catalyseur acide pour obtenir un alkyle 4,6-orthoester de saccharose,
(ii) traitement de l'alkyle 4,6-orthoester de saccharose en conditions acides douces pour obtenir un mélange de 4- et 6-monoesters de saccharose,
(iii) traitement du mélange d'esters par une base afin de convertir le 4-ester de saccharose en 6-ester de saccharose, et
(iv) neutralisation de la solution et isolement du 6-ester de saccharose,
caractérisé en ce qu'une résine échangeuse d'ions macroréticulaire fortement acide est employée à température ambiante pour catalyser la préparation de l'alkyle 4,6-orthoester de saccharose et pour permettre l'hydrolyse de l'étape (ii), et en ce qu'une solution de saccharose et d'un trialkyle orthoester ou d'un cétène acétal dans un solvant organique inerte est passée à travers une colonne de résine macroréticulaire acide afin d'obtenir un éluat contenant un alkyle 4,6-orthoester de saccharose, l'eau étant ajoutée en continu à l'éluat avant de faire passer cette solution aqueuse à travers une seconde colonne de résine échangeuse d'ions acide pour obtenir un mélange de 4- et 6-monoesters de saccharose.

2. Procédé selon la revendication 1, dans lequel la quantité de trialkyle orthoester ou cétène acétal utilisée pendant l'étape (ii) est d'environ 1 à 2 équivalents de moles (ME) par ME de saccharose.

3. Procédé selon la revendication 2, dans lequel la quantité de trialkyle orthoester ou cétène acétal utilisée pendant l'étape (i) est d'environ 1,1 à 1,5 ME par ME de saccharose.

4. Procédé selon l'une quelconque des revendications 1 à 3, dans lequel la base utilisée pour la conversion du 4-ester de saccharose en 6-ester de saccharose est une base soluble dans l'eau ou une base organique.

5. Procédé selon l'une quelconque des revendications 1 à 4, dans lequel le mélange des 4- et 6-monoesters de saccharose ainsi produit est recueilli dans un réservoir, dans lequel est introduit une quantité définie de base soluble dans l'eau pour effectuer la conversion du 4-ester de saccharose en 6-ester de saccharose.

6. Procédé selon l'une quelconque des revendications 1 à 5, dans lequel la base est neutralisée après la conversion du 4-ester de saccharose en 6-ester de saccharose, en faisant passer la solution basique sur une résine échangeuse d'ions acide.

7. Méthode de préparation de sucralose, comprenant les étapes de: préparation d'un 6-ester de saccharose, réaction du 6-ester de saccharose avec un agent de chloruration capable de chlorurer sélectivement les positions 4-, 1'- et 6', préesterification optionnelle du 6-ester de saccharose ainsi produit, déesterification des esters de saccharose et récupération du sucralose, méthode dans laquelle le 6-ester de saccharose a été préparé par:
(i) passage d'une solution de saccharose et de trialkyle orthoester ou d'un cétène acétal dans un solvant organique inerte à travers une résine macroréticulaire fortement acide afin d'obtenir un éluat contenant l'alkyle 4,6-orthoester de saccharose,
(ii) addition continue d'eau à l'éluat et passage du mélange sur une résine échangeuse d'ions acides afin d'hydrolyser l'alkyle 4,6-orthoester de saccharose et pour obtenir un éluat contenant un mélange de 4- et 6-monoesters de saccharose,
(iii) traitement du mélange d'esters par une base afin de convertir le 4-ester de saccharose en 6-ester de saccharose, et
(iv) neutralisation de la solution et isolement du 6-ester de saccharose.
